# EUROPEAN PATENT APPLICATION

(11) **EP 3 583 933 A1**
(43) Date of publication of application: **25.12.2019**
(21) Application number: 18178890.2
(22) Date of filing: 20.06.2018
(51) Int. Cl.: A61K 9/00, A61K 38/22, A61K 9/12

(54) **ADMINISTRATION OF AVIPTADIL BY INHALATION TO TREAT CHRONIC BERYLLIUM DISEASE**

(71) Applicant: Albert-Ludwigs-Universität Freiburg, 79098 Freiburg (DE)
(72) Inventor: MÜLLER-QUERNHEIM, Joachim, 79199 Kirchzarten (DE)
(74) Representative: Kalhammer, Georg

(57) **Abstract**

The present invention relates to kits for use in treating Chronic Beryllium Disease by administering specifically designed aerosols of Aviptadil, whereby the administration of Aviptadil can be obtained by using ultrasonic mesh nebulizers and related kits.

## Description

The present invention relates to the use and kits for treating Chronic Beryllium Disease (CBD) by aerosolised Aviptadil.

Vasoactive intestinal peptide (VIP) is a 28 amino acid polypeptide first isolated from swine duodenum. VIP is a neurotransmitter that is extensively distributed in tissues. VIP exerts diverse actions on the cardiovascular system, pancreas, digestive tract, respiratory system and urological system. The polypeptide derived its name because of its vasodilating action which modifies the intestinal blood flow.

WO 2015/104596 relates to a vasoactive intestinal peptide and its use for switching off and/or preventing harmful and ongoing inflammations. EP 2 152 741 describes peptides with improved properties having the biological activity of vasoactive intestinal peptides and their use for the treatment of lung diseases. WO 03/061680 describes the use of compounds having the biological activity of vasoactive intestinal peptide for the treatment of chronic obstructive pulmonary disease. EP 1 515 745 relates to the use of VIP peptides for the treatment of sarcoidosis. The INN for the vasoactive intestinal peptide having 28 amino acids is "Aviptadil".

Chronic Beryllium Disease (CBD) is a pulmonary granulomatous disorder caused by an immune reaction when individuals are exposed to beryllium. A certain percentage of people who are frequently exposed to Be mainly in their workplace may develop CBD depending on a number of risk factors, in particular their genetic susceptibility, and further factors such as the duration and the concentration of the Be exposure and their smoking habits. It is assumed that the dominant contributor of such risk factors is the genetic susceptibility of the individuals (Petukh et al., PLOS ONE, November 2014, vol. 9, issue 11, 1-11). CBD is also designated as Berylliosis which is a chronic allergic-type lung response caused by exposure to beryllium. It is an incurable occupational lung disease, but symptoms can be treated.

It is an object of the present invention to provide a remedy for Chronic Beryllium Disease. Nebulizers can be used to convert liquids into aerosols at a particle size small enough to be easily inhaled into the lower respiratory tract to reach deep alveolar deposition.

The technical parameters considered important for the lung deposition of drugs are the mass median aerodynamic diameter (MMD - diameter that divides the particle size distribution in half as a function of mass), the fine particle fraction (FPF - fraction of particles with an MMD <5 pm), and the geometric standard deviation (GSD), collectively referred to as "aerosol production parameters".

Aerosols having an MMD of 1 µm to 10 µm are considered to be respirable in humans. The fine particle fraction of particles with an MMAD <5 pm produced by any device depends on both, device and formulation variables. Formulation variables include drug concentration, propellant composition, and cosolvent content. Device variables include valve design, metering volume, spray orifice diameter, and actuator design.

Measurement parameters, physical particle characteristics and setup for testing the preferred embodiment M-neb® dose⁺ mesh nebulizer MN-300/8 and the treatment of patients suffering from CBD with aerosolized Aviptadil are described.

The causative mechanism of many diseases is the over-activity of a biological pathway. A chemical or biological entity that can reduce the activity of the biological pathway can be effective in the prophylaxis and/or treatment of the disease caused by the over activity of the biological pathway. Similarly, the causative mechanism of many diseases is the over production of a biological molecule. A chemical entity that can reduce the production of the biological molecule or block the activity of the over produced biological molecule can be effective in the prophylaxis and/or treatment of the disease caused by the over production of the biological molecule.

Conversely, the causative mechanism of many diseases is the under-activity of a biological pathway. A chemical entity that can increase the activity of the biological pathway can be effective in the prophylaxis and/or treatment of the disease caused by the under activity of the biological pathway. Also, similarly the causative mechanism of many diseases is the under production of a biological molecule. A chemical entity that can increase the production of the biological molecule or mimic the biological activity of the under produced biological molecule can be effective in the prophylaxis and/or treatment of the disease caused by the under production of the biological molecule.

It is an object of the present invention to provide a suitable medicament for the prophylaxis and/or treatment of Chronic Beryllium Disease (CBD).

The present invention is defined by the claims. Further advantageous features, aspects and details of the invention are evident from the description and the examples of the present application.

### Detailed Description of the invention

The present invention relates to Aviptadil which is highly biologically and pharmacologically active as therapeutic for chronic lung disorders. Aviptadil which can be used according to the invention for the treatment of said disease is a ligand which can occupy certain receptors and by doing so induces cellular processes which play important roles in connection with biological events underlying the disease, and restoring the physiologically normal, healthy situation. Aviptadil is delivered to the patients in need by aerosols of certain physical size and at certain pharmaceutical strength, thus targeting precisely the correct receptors at the correct location within the lungs at the right time for best possible biological effects. This is achieved in a preferred embodiment by specifically tailored aerosol characteristics provided by the ultrasonic mesh nebulizer M-neb® dose⁺ MN-300/8.

It has been shown that Aviptadil can be successfully used for treatment of CBD. Furthermore, the present invention discloses pharmaceutical compositions and precise inhalation delivery methods useful for treatment of said disease within said methods.

### Chronic Beryllium Disease (CBD)

Beryllium (Be) is an alkaline earth metal. Be has unique properties such as strength, electrical and thermal conductivity, high melting point, and resistance to corrosion, which makes the use of the metal and its oxide attractive in a wide range of important technological applications.

Workplaces and products with potential Be exposure and usage can be summarized as follows:

| | | |
|---|---|---|
| - Additives to glass, ceramic, plastics | - Golf clubs | - Pen clips |
| | - Gyroscopes | - Personal computers |
| - Aerospace industries (e.g. aircraft frames, engines, and brakes) | - Metallurgic industries/recycling | - Precision instruments |
| | | - Recycling workplaces |
| | - Microelectronics | - Satellites |
| - Automobile industries (engines, electronic parts) | - Microwave devices | - Springs |
| | - Military vehicle armour | - Structural material in space technology |
| - Brass alloys | - Mirrors | |
| - Camera shutters | - Missile production and maintenance | - Submarine cable housings |
| - Ceramic industries | | |
| - Chemical industries | - Missile guidance systems | - Transistor mountings |
| - Dental workshops | - Non-sparking tools | - Wheels |
| - Electronic industries | - Nuclear reactors and industries | - X-ray tubes |
| - Fluorescent lamp production/disposal | | |
| | - Optical industries/workshops | |
| - Gems | | |

The recognition of lung disease in association with Be exposure occurred in the 1940s. The chronic lung disease was first described among workers exposed to Be-containing materials used in the manufacture of fluorescent lamps. In primary production of Be, which was used in nuclear weapons components, severe dermatitis, pneumonitis, and chronic granulomatous lung disease were identified. Today, CBD is officially classified as a lung disease due to external agents.

Most exposures to Be that cause disease are related to some aspect of Be processing. The major pathway for human exposure is through airborne particles of Be metal, alloys, oxides, and ceramics. Be particles are inhaled into the lower respiratory tract and reach the paraenchyma of the lung. Exposures not directly related to inhalation of workplace air, such as hand-to-mouth exposure, dermal contact with ultrafine particles, and resuspension following deposition of beryllium dust onto clothing may also occur.

The understanding of CBD has significantly evolved over the last few years. Tests performed on peripheral blood and bronchoalveolar lavage (BAL) mononuclear cells extracted from CBD patients, have shown a dose dependent proliferation of specific T cell clones in response to *in vitro* Be exposure. Cells from healthy control subjects or patients with other granulomatous disorders did not change their proliferation rate when exposed to Be salts. Based on these facts, a test called beryllium lymphocyte proliferation test (BeLPT) is currently used as the only available diagnostic tool to detect Be sensitization as a necessary but not sufficient criterion to diagnose CBD. A genetic predisposition to develop CBD has been identified. Several genetic variants of the major histocompatibility complex HLA-DPb1 were found in 97% of CBD. CBD develops insidiously with symptoms of dyspnea on exertion, cough, fatigue, chest pain, weight loss, night sweats, fever, and anorexia. Chest radiographs range from normal to small nodular opacities with an upper level predominance up to formation of conglomerate masses. Mediastinal and hilar lymphadenopathy is present in approximately one third of the individuals examined by chest radiograph or computed tomography. In the early stages of the disease, alterations in lung function include airflow obstruction, later developing a mixed pattern of obstruction and restriction, or pure restriction toward the end stage of the disease. Gas exchange abnormalities are particularly notable, especially during exercise.

The released proinflammatory cytokines amplify the autoaggressive immune response, resulting in formation of mononuclear cell infiltrates and nonnecrotizing granulomas in target organs where Be has deposited. The histopathology includes a combination of mononuclear cell infiltration and typical nonnecrotizing granulomas with varying degrees of fibrosis.

Aviptadil is a widely distributed human neuropeptide which mediates a variety of physiological responses including gastrointestinal secretion, relaxation of gastrointestinal vascular and respiratory smooth muscle activities, immune cell regulation, and apoptosis. Under physiologic conditions Aviptadil acts as a neuroendocrine mediator. The biological effects are mediated via specific receptors (VIP-R) located on the surface membrane of various cells. Aviptadil has vascular smooth muscle relaxant activity, positive inotrope effect on the heart, sustained bronchodilation activity without remarkable cardiovascular side effects, and is effective against disorders or diseases relating to bronchial spasms including asthma, impotence, ischaemia, dry eye, chronic inflammatory response syndrome (CIRS) and mental disorders, such as Alzheimer's disease.

Aviptadil receptors have been detected on airway epithelium of the trachea and the bronchioles, in macrophages surrounding capillaries, in connective tissue of trachea and bronchi, in alveolar walls, and in the subintima of pulmonary veins and pulmonary arteries. Peptidergic nerve fibers are considered the source of Aviptadil in the lungs. Aviptadil decreases the resistance in the pulmonary vascular system.

Still another aspect of the present invention relates to the use of Aviptadil as an active ingredient, together with at least one pharmaceutically acceptable carrier, excipient and/or diluents for the use of a pharmaceutical composition in the treatment and/or prophylaxis of Chronic Beryllium Disease (CBD).

Such pharmaceutical compositions comprise Aviptadil as an active ingredient, together with at least one pharmaceutically acceptable carrier, excipient, binders, disintegrates, glidents, diluents, lubricants, coloring agents, sweetening agents, flavoring agents, preservatives or the like. The pharmaceutical compositions of the present invention can be prepared in a conventional solid or liquid carrier or diluents and a conventional pharmaceutically-made adjuvant at suitable dosage level in a known way.

Aviptadil for use in the treatment of CBD is provided in a form suitable for administration by inhalation.

Aviptadil is a peptide which may form pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogen-benzenesulfonic acid, picric acid, adipic acid, D-o-tolyltartaric acid, tartronic acid, a-toluic acid, (o, m, p)- toluic acid, naphthylamine sulfonic acid, and other mineral or carboxylic acids well known to those skilled in the art. The salts are prepared by contacting the free base form with a sufficient amount of the desired acid to produce a salt in the conventional manner.

The aerosol which is used according to the present invention for the treatment of CBD comprises droplets, which are small enough to be easily inhaled and such liquid droplets have a certain diameter which ranges from about 0.5 to about 10 µm, preferably from about 2.0 to about 6.0 µm and especially preferred between about 2.8 and 4.5 µm. Those liquid droplets are finely dispersed within a carrier gas. As suitable carrier gas inert gases such as helium, neon or argon can be used. Preferably, however, inert gases which are easily available like nitrogen (N₂) or carbon dioxide (CO₂) are used. It is also possible to use air, whereby the oxygen content may be reduced.

In an alternative embodiment the Aviptadil is used in the form of dry particles which have a diameter of about 2.0 to 4.0 µm whereby about 90% of the particles are within said range.

The aerosol droplets comprise the biologically active agent Aviptadil in a concentration of about 20 to 200 µg Aviptadil/ml aerosol, preferably from 35 to 140 µg Aviptadil/ml aerosol and particularly preferred between 60 and 80 µg Aviptadil/ml aerosol.

The characterization of the aerosol regarding droplet diameter and content of Aviptadil can be easily performed by measurement devices known to the person skilled in the art.

In a preferred embodiment the aerosol is produced by an ultrasonic mesh nebulizer whereby a particularly preferred embodiment is the M-neb® dose⁺ MN-300/8. Alternatively, however, the aerosol can be produced by commercially available inhalers which meet the requirement of providing an aerosol having the defined size of the droplets.

Another aspect of the present invention is a dosage regimen whereby the aerosol is applied to a patient in need thereof suffering from Chronic Beryllium Disease by applying 4 doses daily whereby each dose comprises 70 µg Aviptadil. In a particularly preferred embodiment the dosage of Aviptadil is 280 µg which is applied by four 4 doses whereby each dose comprises about 70 µg Aviptadil. In a particularly preferred embodiment 2 doses are applied in the morning and 2 doses a 70 µg Aviptadil are applied in the evening.

The present invention relates to the use of an aerosol comprising Aviptadil in the prophylaxis and/or treatment of Chronic Beryllium Disease comprising administering to a patient in need thereof a suitable dose of Aviptadil according to the present invention.

Accordingly, the terms "prophylaxis" or "treatment" includes the administration of the peptidic entity of the present invention to prevent, inhibit, or arrest the symptoms of Chronic Beryllium Disease. In some instances, treatment with Aviptadil will be done in combination with other protective compounds to prevent, inhibit, or arrest the symptoms of Chronic Beryllium Disease.

The term "active agent" or "therapeutic agent" as used herein refers to an agent that can prevent, inhibit, or arrest the symptoms and/or progression of Chronic Beryllium Disease.

The term "therapeutic effect" as used herein, refers to the effective provision of protection effects to prevent, inhibit, or arrest the symptoms and/or progression of Chronic Beryllium Disease.

The term "a therapeutically effective amount" as used herein means a sufficient amount of Aviptadil to produce a therapeutic effect, as defined above, in a subject or patient in need of treatment.

The terms "subject" or "patient" are used herein to mean any mammal, including but not limited to human beings, including a human patient or subject to which the compositions of the invention can be administered.

Aviptadil can be used for the prophylaxis and/or treatment of Chronic Beryllium Disease in combination administration with another therapeutic compound. As used herein the term "combination administration" of a compound, therapeutic agent or known drug with Aviptadil means administration of the drug and the one or more compounds at such time that both the known drug and Aviptadil will have a therapeutic effect. In some cases this therapeutic effect will be synergistic. Such concomitant administration can involve concurrent (i.e. at the same time), prior, or subsequent administration of the drug with respect to the administration of Aviptadil.

Without wishing to be bound by a theory it is assumed that Aviptadil has therapeutic activity based on any one of the following activities listed in a) to g).
a) Aviptadil could inhibit the activity of an overly active biological pathway;
b) Aviptadil could inhibit the production of an overly produced biological molecule;
c) Aviptadil could inhibit the activity of an overly produced biological molecule;
d) Aviptadil could increase the activity of an under-active biological pathway;
e) Aviptadil could increase the production of an under-produced biological molecule;
f) Aviptadil could mimic the activity of an under-produced biological molecule; or
g) Aviptadil could prevent, inhibit, or arrest the symptoms and/or progression of Chronic Beryllium Disease.

As used herein "inhibition" is defined as a reduction of the activity or production of a biological pathway or molecule activity of between 10 to 100%. More preferably the reduction of the activity or production of a biological pathway or molecule activity is between 25 to 100%. Even more preferably the reduction of the activity or production of a biological pathway or molecule activity is between 50 to 100%.

As used herein "increase" is defined as an increase of the activity or production of a biological pathway or molecule of between 10 to 100%. More preferably the increase of the activity or production of a biological pathway or molecule activity is between 25 to 100%. Even more preferably the increase of the activity or production of a biological pathway or molecule activity is between 50 to 100%.

In more specific embodiments, aerosolized Aviptadil is administered in doses ranging from about 140 µg to 560 µg per day given in the M-neb® dose⁺ mesh nebulizer MN-300/8 three to four times per day with overnight break. In a preferred embodiment, a dose of 280 µg per day given in a M-neb® dose⁺ mesh nebulizer MN-300/8, whereby suitable doses are administered four times per day preferably with overnight break.

In another aspect, the invention features a method of accurately determining respiratory deposition of Aviptadil administered by aerosol delivery. This technology is unique and applies to the delivery of Aviptadil to patients with Chronic Beryllium Disease, and will become apparent to the person skilled in the art from a review of the ensuing detailed description taken in conjunction with the following results. The specific breathing by using slow and deep inspiration describes a reduction in inspiratory flow and a greatly prolonged inspiratory time. The slow inspiration allows aerosol particles to bypass the upper airways thus making them available for deposition in the lung. The prolonged inspiration allows for suitable settling of aerosols in the lung periphery. The prolongation of the inspiratory time and the advanced settling promotes inspiratory deposition before remaining particles can be exhaled. It is possible under these circumstances to have almost 100% of the inhaled particles depositing before exhalation begins. Diseases of the lung parenchyma result in geometric changes in the lung periphery that can minimize the deposition of inhaled particles.

The following examples are put forth so as to provide those of ordinary skill in the art with a complete disclosure and description of how to make and use the therapeutic methods of the invention and compounds and pharmaceutical compositions, and are not intended to limit the scope of what the inventors regard as their invention.

### Methods for generating Aviptadil aerosols

In chronic lung diseases, pulmonary administration offers a number of advantages compared to the systemic oral and parenteral (injections) routes:
a) direct targeting of the diseased organ
b) rapid onset of drug's biological activity
c) reduction of potentially negative systemic side effects by local metabolism
d) prevention of the first pass through the liver
e) more precise dosing possible
f) higher local dosages possible
g) avoiding of subcutaneous or intravenous injections
h) reduction of dose and costs
i) reduction of potentially adverse effects by lowering total dose.

The devices used for inhaled drug delivery are collectively referred to as orally inhaled drug products, encompassing inhalers (metered-dose, dry powder and aqueous droplet), and nebulizers (jet, ultrasonic and vibrating mesh). The present invention relates in preferred embodiment to the use of a specific ultrasonic mesh nebulizer M-neb® dose⁺

MN-300/8 can be used to deliver Aviptadil to the lungs. Ultrasonic nebulizer use electricity to vibrate a piezoelectric crystal at high frequency. The resultant vibrations are transmitted to a reservoir containing the liquid peptidic drug, creating a series of waves from which liquid droplets separate to form an aerosol. Mesh nebulizers use the ultrasonic principle to generate droplets which are then pushed through a static or vibrating mesh or plate (either electro-formed or laser drilled) to form an aerosol cloud prior to inhalation. The M-neb® dose⁺ MN-300/8 can incorporate sensing devices to detect the patient's inspiration in order to provide breath activated or breath integrated systems.

The ultimate responsibility for the safety, quality and efficacy of inhaled drugs and medical devices lies with the regulatory bodies designated to safeguard public health. In European Union and in the USA this function is performed by the European Medicines Agency (EMA - and the local country agencies) and by the Food and Drug Administration (FDA).

The regulatory authorities are supported in this role by the Pharmacopoeias whose job is to define the standards with which the drug formulation shall comply and the methods by which compliance will be adjudged, and by the International Standards Organization (ISO) whose function is to define the standards and methods relating to the medical device, e.g. inhaler, nebulizer, concerned. Two of the main factors largely recognized as Critical Quality Attributes (CQAs) in the testing of orally inhaled and nasal drug products (in both pharmaceutical development and batch release) are: Emitted dose (the total amount of drug delivered from the drug device and hence available to the patient) and Particle size (aerodynamic size distribution of the particles or droplets that make up the emitted aerosol cloud.) Particle size determines the percentage of the total emitted dose that actually reaches the lungs during inhalation and is therefore therapeutically effective. These two physical tests form the basis of the parameters used by the regulators to characterize inhalation products.

The preferred instrument of choice for measuring the Aerodynamic Particle Size Distribution of inhaled products is the cascade impactor. Cascade impactors measure aerodynamic particle size; active pharmaceutical ingredient; and the entire dose. Cascade impactors are precision instruments that separate a sample on the basis of particle inertia (function of velocity and aerodynamic particle size) without the need to know either particle density or shape.

The multi-stage cascade impactor is the instrument of choice for the particle size characterization of aerosols from portable inhalers. Several different inhalers can be used in order to provide the aerosol. Pressured metered dose inhalers with or without spacer/valved holding chamber, nebulizer or aqueous nasal spray pumps may be used.

Such spray pumps must be able to provide an aerosol whereby the Aptidavil containing droplets have a diameter below 10 µm. This can be achieved for example by using special, portable electromechanical pumps which can provide sufficiently small droplets.

The aerodynamic size distribution of an aerosol cloud define where the particles in that cloud are likely to deposit following inhalation. It is generally accepted, that to be therapeutically effective, the particles should be in the range of 1 to 5 micrometers in order to deposit in the lungs. Particles having an aerodynamic size in excess of 5.5 micrometers will generally impact in the oropharynx and be swallowed whereas below 1 micrometer the possibility exists that the particles will remain in the air stream and be exhaled. The results obtained and measured were performed with a Next Generation Impactor (NGI).

This NGI comprises the following features:
1) designed and accepted by pharma and biotech industry for inhaler testing;
2) meets and exceeds all European and US Pharmacopoeia specifications;
3) particle size range between 0.24 - 11.7 micrometers (dependent on flow rate);
4) comprises 7 stages, 5 with cut-offs between 0.54 - 6.12 micrometers at flow rates from 30 to 100 L/min;
5) excellent stage efficiency, accuracy and reproducibility;
6) calibrated flow rate range from 30 - 15 100 L/min;
7) additional calibration at 15L/min for nebulizer applications;
8) supplied with full stage mensuration report (system suitability);
9) low inter-stage wall losses ensure good drug recovery (mass balance);
10) user-friendly design for maximum throughput and easy automation;
11) electrically conductive and uneffected by static;
12) Design and calibration features are formally documented and published.

The impactor itself comprises three main parts:
a) The cup tray containing the eight collection cups used to collect the samples prior to analysis.
b) The bottom frame used to support the cup tray.
c) The lid containing the inter-stage passageways and the seal body which holds the nozzles in place.

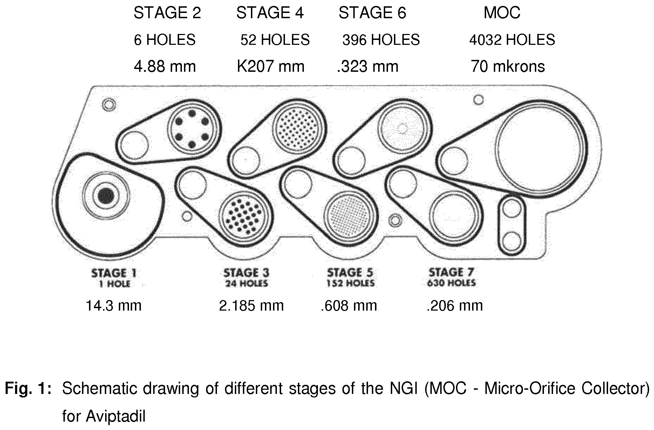

The different stages of a next generation impactor are shown schematically in Figure 1.

Aviptadil was tested for the activity as a therapeutic agent for the prophylaxis and/or treatment of Chronic Beryllium Disease. Furthermore, the present invention relates to the use of Aviptadil as pharmaceutically active agent in medicine, i.e. as medicaments. As used herein, the term "Aviptadil" shall also refer to salts, enantiomers, diastereomers, racemates, prodrugs and hydrates of Aviptadil. Aviptadil may be administered to a subject in need thereof, e.g. a human individual, in pharmaceutical compositions comprising also other pharmaceutically effective compounds, such as e.g. epoprostenol, iloprost, treprostinil, uniprost, bosentan, ambrisentan, macitentan, selexipag, sildenafil, tadalafil, riociguat, diltiazem, ramipril, atorvastatin, imatinib, interferon gamma, pirfenidone, nintedanib or_antibodies e.g. against alpha v beta 6 integrins.

It is likely that the therapy with Aviptadil, alone or in combination with the above-mentioned substances, may lower existing but undesired drug effects in a subject in need of those drugs.

### EXAMPLES

The peptidic entities of the invention were tested for activity as described in the following examples.

For the precise measurement of the particle size diameter in the experiments the M-neb® dose⁺ mesh nebulizer MN-300/8 was used.

For commercial purposes inhalers or nebulizers, respectively, can be used which are designed according to the Guidelines on the Pharmaceutical Quality of Inhalation and Nasal Products provided by the European Medicines Agency. It is important that all requirements with regard to product safety and hygienic requirements are observed. The inhalers are frequently used for the therapy of lung diseases like asthma or other allergic reactions. It is important that the aerosol comprising droplets (dry or liquid) have a diameter of about 0.5 up to about 10.0 µm whereby, however, diameters between about 2.0 to about 6.0 µm are preferred. The aerosol consists of the liquid or dry droplets or particles, respectively, and the carrier gas which may be preferably an inert gas like helium or N2. Even pressurized air can be used whereby, however, care has to be taken that no undesired oxidation occurs due to the presence of oxygen.

In a preferred embodiment the nebulizers or inhalers have a dosing device whereby a certain volume of aerosol is provided. In a preferred embodiment the doses contain 60 to 80 µg Aviptadil, preferably about 70 µg Aviptadil. When the dose comprises about 70 µg Aviptadil it is easily possible to deliver about 280 µg Aviptadil per day by applying two doses comprising 70 µg Aviptadil preferably in the morning and two doses comprising about 70 µg Aviptadil each in the evening of each day.

### Example 1

Using the M-neb® dose⁺ mesh nebulizer MN-300/8 and the respective mouthpiece, Aviptadil has been tested with the COPLEY next generation impactor (NGI). The mass median aerodynamic diameter (MMD) of Aviptadil dissolved in 0.9% NaCI was 3.3 - 3.5 µm per emitted particle. The number of those particles <5 µm = 85.70%, and the dose delivered at the mouthpiece was 90.2% of the tested dosages.

Aviptadil has been tested in 0.9% NaCl solution at different drug concentrations (35 µg/ml, 70 µg/ml, 140 µg/ml, 200 µg/ml, 250 µg/ml, 400 µg/ml). Results show an excellent linearity for the tested peptidic drugs when measuring the aerosol output rates at the mouthpiece over increasing numbers of breathing cycles.

### Example 2

Aviptadil has been tested in 0.9% NaCl solution at different drug concentrations (35 µg/ml, 70 µg/ml, 140 µg/ml, 200 µg/ml, 250 µg/ml, 400 µg/ml). Results show that the respective biological activity is best beween 35 µg/ml - 140 µg/ml.

### Example 3

Aviptadil has been tested in 0.9% NaCl solution at different time points over increasing numbers of breathing cycles. Diseases of the lung parenchyma result in geometric changes in the lung periphery that can minimize the deposition of inhaled particles. The specific breathing by using slow and deep inspiration allows aerosol particles to bypass the upper airways thus making them available for deposition in the lung. The prolonged inspiration allows for suitable settling of aerosols in the lung periphery. The prolongation of the inspiratory time and the advanced settling promotes inspiratory deposition before remaining particles can be exhaled. It is possible under these circumstances to have almost 100% of the inhaled particles from the mouthpiece depositing before exhalation begins. Inhalation times between 10 min to 15 min are much better than short times of inhalation between 2-4 min per treatment.

### Example 4

A patient who was exposed to Beryllium (Be) at his workplace, developed Chronic Beryllium Disease (CBD), which was diagnosed by tests performed on his peripheral blood and bronchoalveolar lavage (BAL) mononuclear cells. These tests have shown a dose dependent proliferation of specific T cell clones in response to *in vitro* Be exposure. Cells from healthy control subjects or patients with other granulomatous disorders do not change their proliferation rate when exposed to Be salt. Based on these facts, a test called beryllium lymphocyte proliferation test (BeLPT) added the mandatory diagnostic criterion of Beryllium-sensitization for the diagnosis of CBD for the patient. Due to the lack of available treatment options for CBD, the patient qualified for the Early Access Program treatment with inhaled vasoactive intestinal peptide. Therapy was initiated at a dose of 4 x 70 µg/ml per day dosage (280 µg per day with overnight break). After 6 months of treatment, the cytokine production (TNF-alpha, IL-17 - key players in the onset of the disease) was significantly diminished in the patient.

It has been observed that by applying aerosolized Aviptadil to patients suffering from Chronic Beryllium Disease at least the further worsening of the disease could be blocked.

## Claims

1. Aerosol comprising droplets having a particle size small enough to be easily inhaled for use in the treatment of Chronic Beryllium Disease.

2. Aerosol for use according to claim 1 comprising droplets having a diameter of about 0.5 to 10 µm containing Aviptadil within a carrier gas.

3. Aerosol for use according to claim 2 **characterized in that** at least 80% of the droplets have a diameter between 2.0 and 6.0 µm.

4. Aerosol for use according to claim 2 **characterized in that** the droplets have a diameter between 2.8 and 4.5 µm .

5. Aerosol for use according to any of claims 1-4 **characterized in that** it comprises 35 µg to 140 µg Aviptadil/ml aerosol.

6. Aerosol for use according to any of claims 1-4 **characterized in that** it comprises 60 µg to 80 µg Aviptadil/ml aerosol.

7. Aerosol for use according to any of claims 1-6 **characterized in that** the droplets are liquid.

8. Aerosol according to any of claims 1-7 **characterized in that** the aerosol is provided by an ultrasonic mesh nebulizer.

9. Aerosol according to claim 8 **characterized in that** the ultrasonic mesh nebulizer is M-neb® dose⁺ MN-300/8.

10. Aerosol according to any of claims 1-7 **characterized in that** the aerosol is produced by an inhaler.

11. Aerosol according to any of claims 1-10 for use in the treatment of Chronic Beryllium Disease by inhalative administration of 2-4 doses comprising 70 µg Aviptadil each 2-4 times a day.

12. Aerosol for use according to claim 11 **characterized in that** the daily dose of Aviptadil amounts to 280 µg Aviptadil.

13. Pharmaceutical kit which provides aerosolized Aviptadil according to any of claims 1-12.

14. Pharmaceutical kit according to claim 13 **characterized in that** it is a nebulizer.

15. Pharmaceutical kit according to claim 13 **characterized in that** it is an inhaler.
